# EUROPEAN PATENT SPECIFICATION

(11) **EP 0 964 023 B1**
(45) Date of publication and mention of the grant of the patent: **14.11.2001**
(21) Application number: 99111257.4
(22) Date of filing: 10.06.1999
(51) Int. Cl.: C08L 83/04, A61K 7/48, A61K 7/02

(54) **Silicone oil emulsion, compositions, and method of manufacture**
Silikondispersion,Zusammensetzung und Verfahren zur Herstellung
Dispersion de siloxane, composition et procédé de préparation

(30) Priority: 10.06.1998 JP 17811698
(43) Date of publication of application: 15.12.1999
(73) Proprietor: Dow Corning Toray Silicone Company, Ltd., Chiyoda-ku, Tokyo 100-0005 (JP)
(72) Inventor: Morita, Yoshitsugu, Main Office for R & D, 2-2, Chigsa Kaigan, Ichihara-shi, Chiba (JP); Kobayashi, Kazuo, Main Office for R & D, 2-2, Chigsa Kaigan, Ichihara-shi, Chiba (JP); Tachibana, Ryuji, Main Office for R & D, 2-2, Chigsa Kaigan, Ichihara-shi, Chiba (JP)
(74) Representative: Sternagel, Fleischer, Godemeyer & Partner Patentanwälte

(56) References cited:
- EP-A- 0 501 791
- EP-A- 0 787 758
- EP-A- 0 829 253
- EP-A- 0 917 870
- WO-A-97/32561
- US-A- 4 980 167

## Description

This invention is directed to a silicone dispersion, a method of manufacturing thereof, a silicone composition made from said silicone dispersion, and a method for manufacturing said silicone composition as well as a lubricating agent and a cosmetic formulation containing said silicone composition.

More specifically, the invention is a silicone dispersion that contains crosslinked silicone particles in silicone oil droplets which are dispersed in water; a method of manufacturing the aforementioned silicone dispersion; a method for the preparation of a silicone composition therefrom; a silicone composition obtainable therefrom comprising crosslinked silicone particles uniformly dispersed in a silicone oil and use of said silicone composition as lubricating agent or in cosmetic formulations.

A general representation of the prior art is provided by reference to the following documents JP-As 01-081856; 02-243612 (US-A 4,986,167); 03-271211; 09-053047 (US-A 5,760,109); 63-152308; 01-165509; 01-207354; 02-043263 (US-A 4,987,169); 06-502646 (US-A 5,154,849) and 07-330537. However, in silicone compositions prepared by the above methods, cross-linked silicone particles are dispersed in silicone oil nonuniformly, and therefore they cannot demonstrate their specific properties sufficiently.

It is thus an object of the present invention to provide a silicone oil dispersion that contains cross-linked silicone particles in silicone droplets which are dispersed in water; a method of manufacturing the silicone oil dispersion; and a method for the preparation of a silicone composition made from said silicone oil dispersion resulting in a silicone composition comprising cross-linked silicone particles uniformly dispersed in a silicone oil.

This object has been attained by a silicone dispersion comprising crosslinked silicone particles which have an average particle diameter of 0.1 to 100 µm, the crosslinked silicone particles being contained in silicone oil droplets having an average diameter of more than 0.1 to 500 µm, the silicone oil droplets containing the crosslinked silicone particles being dispersed in water, the diameter of the crosslinked silicone particles being smaller than the diameter of the silicone oil droplets.

Said silicone dispersion containing crosslinked silicone particles of an average particle diameter of 0.1 to 100 µm in silicone oil droplets of an average droplet diameter of more than 0.1 to 500 µm and wherein the diameter of the crosslinked silicone particles is smaller than the diameter of the silicone oil droplets, are prepared by a method comprising:
a) dispersing in water a crosslinkable silicone composition containing crosslinkable silicon species and a non-crosslinkable silicone oil in an amount which exceeds the amount of non-crosslinkable silicone oil which can be held by the crosslinked product resulting from the crosslinkable silicon species; and
b) affecting crosslinking of the crosslinkable silicone composition.

Preferably the amount of silicone oil is within the range of 200-5000 parts by weight per 100 parts by weight of the crosslinkable silicon species.

The above object is further attained by a method for the preparation of a silicone composition containing cross-linked silicone particles with an average diameter of 0.1 to 100 µm which are uniformly dispersed in a silicone oil, the method comprising removing water from a silicone dispersion as defined above and a silicone composition obtainable thereby.

These and other features of the invention will become apparent from a consideration of the detailed description.The following is a more detailed description of a silicone oil dispersion of the present invention.

A dispersion of the present invention contains crosslinked silicone particles in silicone oil droplets which are uniformly dispersed in water. The crosslinked silicone particles contained in the dispersion of the present invention are obtained by crosslinking a crosslinkable composition. This crosslinking can by preferably affected by a hydrosylilation reaction, a condensation reaction, by organoperoxide type initiators, or by exposure to high-energy rays. The most preferred crosslinking reaction is the hydrosylilation or the condensation reaction.

There are no special limitations with regard to the silicone oil used for the formation of the silicone oil droplets according to the present invention. The silicone oil may have a completely linear, partially-branched, cyclic, or a branched-chain molecular structure. The most preferred structure is linear or cyclic. It is preferred that the silicone oil does not participate in the cross-linking reaction during formation of the cross-linked silicone particles. For example, when the crosslinked silicone particles are formed by hydrosylilation the silicone oil does not contain alkenyl groups or silicon-bonded hydrogen atoms. For example the silicone oil can be a dimethylpolysiloxane with terminal trimethylsiloxy groups, a methylphenylpolysiloxane with terminal trimethylsiloxy groups, a copolymer of methylphenylsiloxane and dimethylsiloxane with terminal trimethylsiloxy groups, a copolymer of methyl (3,3,3-trifluoropropyl) siloxane and dimethylsiloxane with terminal trimethylsiloxy groups, a cyclic dimethylsiloxane, or a cyclic methylphenylsiloxane. When the cross-linked silicone particles are formed by a condensation reaction, it is preferred that the oil does not contain silanol groups, silicon-bonded hydrogen atoms, or silicon-bonded hydrolyzable groups. For example, the silicone oil can be an oil similar to those mentioned above, i.e., a dimethylpolysiloxane with terminal dimethylvinylsiloxy groups, or a copolymer of methylvinylsiloxane and dimethylsiloxane with terminal dimethylvinylsiloxy groups, a methylvinylpolysiloxane with terminal trimethylsiloxy groups, or a cyclic methylvinylsiloxane. It is preferred that the silicone oil of the present invention has a viscosity of 1 to 100,000,000 mPa·s, preferably 2 to 10,000,000 mPa·s, at 25°C.

The average diameter of silicone oil droplets in the dispersion of the present invention is within a range of more than 0.1 to 500 µm, preferably within a range of 0.5 to 200 µm. This is because an dispersion with droplets of an average diameter beyond this range will have low stability.

The average diameter of cross-linked silicone particles in the dispersion of the present invention is within a range of 0.1 to 100 µm, preferably within a range of 0.5 to 50 µm. This is because an dispersion with particles of an average diameter beyond this range will have low stability. In the dispersion of the present invention, the diameter of the cross-linked silicone particles is smaller than the diameter of the silicone oil droplets. The cross-linked silicone particles may have a spherical, elongated, flat, or irregular shape. A spherical shape is preferred.

The dispersion of the present invention can be used as a starting material for the preparation of cosmetic substances. By removing water from the dispersion, it is possible to obtain a liquid, cream-like, paste-like, or a grease-like silicone composition having cross-linked silicone particles uniformly dispersed in silicone oil.

The following is a description of a method for manufacturing the silicone dispersion of the present invention. A cross-linkable silicone composition according to the present invention may be crosslinked by a hydrosylilation reaction, a condensation reaction, by action of organoperoxide initiators, or by irradiating the composition with high-energy rays. The most preferred reaction is a hydrosylilation reaction or a condensation reaction.

A suitable silicone composition that is crosslinkable by a hydrosilylation reaction comprises an organopolysiloxane having in its molecule at least two alkenyl groups, an organopolysiloxane having in its molecule at least two silicone-bonded hydrogen atoms, and a hydrosylilation catalyst.

Alkenyl groups in the first-mentioned organopolysiloxane component are preferably selected from vinyl groups, allyl groups, butenyl groups, pentenyl groups, and hexenyl groups. The most preferred alkenyl group is vinyl. Groups other than alkenyl groups contained in the first-mentioned organopolysiloxane include monovalent hydrocarbon groups like alkyl groups such as methyl groups, ethyl groups, propyl groups, butyl groups; cycloalkyl groups such as cyclopentyl groups, cyclohexyl groups; aryl groups such as phenyl groups, tolyl groups, xylyl groups; aralkyl groups such as benzyl groups, phenethyl groups, 3-phenylpropyl groups; or halogenated hydrocarbon groups such as 3-chloropropyl groups, 3,3,3-trifluoropropyl groups. These organopolysiloxanes may have a linear, cyclic, branched, or partially-branched molecular structure. To form elastomer-like, cross-linked silicone particles, the linear and partially-branched structures are preferred. Although there are no special limitations with regard to the viscosity of this first organopolysiloxane, it should not limit the dispersing of the cross-linkable silicone composition in water. It is preferred that at 25°C, the viscosity be within a range of 20 to 100,000 mPa·s, preferably 20 to 10,000 mPa·s.

Examples of groups other than hydrogen atoms in the second-mentioned organopolysiloxane are the same as the above monovalent hydrocarbon groups. The second-mentioned organopolysiloxane may have a linear, cyclic, branched, or partially-branched structure. Although there are no special limitations with regard to viscosity of this second organopolysiloxane, it should not limit dispersing of the crosslinkable silicone composition according to the present invention in water. It is preferred that the viscosity is within a range of 1 to 10,000 mPa·s at 25 °C. This second organopolysiloxane ist used in the crosslinkable silicone composition in an amount sufficient for curing the composition, preferably in an amount of 0.3 to 200 parts by weight per 100 parts by weight of the first organopolysiloxane.

The hydrosylilation reaction catalyst contained in the cross-linkable silicone composition of the present invention is preferably a platinum catalyst such as chloroplatinic acid, an alcoholic solution of chloroplatinic acid, an olefin complex of platinum, an alkenylsiloxane complex of platinum, platinum black, or platinum on a silica carrier.. It is preferred to use an aqueous dispersion containing the hydrosylilation catalyst with an average particle diameter not exceeding 1 µm in combination with our crosslinkable silicone composition. The exact order of mixing or combining said catalyst and said silicone composition is not important. For example a crosslinkable silicone composition that contains the hydrosylilation catalyst in water can be prepared by dispersing in water the crosslinkable silicone composition which has been premixed with the hydrosylilation catalyst, or the catalyst can be added to water after dispersing it in the catalyst-free cross-linkable silicone composition. It is preferred that the hydrosylilation catalyst is used in our crosslinkable composition in an amount sufficient for accelerating the crosslinking of the crosslinkable composition. For example, when a platinum-system catalyst is used as hydrosylilation catalyst, it is normally used in an amount of 1 x 10⁻⁷ to 1 x 10⁻³ parts by weight of platinum metal per 100 parts by weight of the first organopolysiloxane.

A silicone composition that is crosslinkable by a condensation rection may comprise, an organopolysiloxane containing at least two hydrolyzable groups such as aminoxy, acetoxy, oxime, alkoxy or hydroxyl group; a silane crosslinking agent having at least three hydrolyzables group such as aminoxy, acetoxy, oxime or alkoxy; and a condensation reaction catalyst such as an organic titanium compound or an organic tin compound.

Alkoxy groups contained in said organopolysiloxane are preferably selected from methoxy groups, ethoxy groups, and methoxyethoxy groups. Oxime groups contained in said organopolysiloxane are preferably selected from dimethylketoxime groups and methylethylketoxime groups. Other groups that can be contained in the organopolysiloxane include monovalent hydrocarbon groups like alkyl groups such as methyl groups, ethyl groups, propyl groups, butyl groups; cycloalkyl groups such as cyclopentyl groups, cyclohexyl groups; aryl groups such as phenyl groups, tolyl groups, xylyl groups; aralkyl groups such as benzyl groups, phenethyl groups, 3-phenylpropyl groups; or halogenated hydrocarbon groups such as 3-chloropropyl groups, 3,3,3-trifluoropropyl groups. The organopolysiloxane may have a linear, cyclic, branched, or a partially-branched molecular structure. The linear or partially-branched molecular structure is preferable for the formation of elastomer-like crosslinked silicone particles. Although there are no special limitations with regard to the viscosity of the organopolysiloxane, it should not limit the dispersing of the crosslinkable silicone composition in water. It is preferred that the viscosity is within a range of 20 to 100,000 mPa·s, preferably between 20 and 10,000 mPa·s. at 25°C.

Oxime groups and alkoxy groups contained in the silane cross-linking agent may be the same as those exemplified above. The silane crosslinking agent is preferably selected from methyltrimethoxysilane. vinyltrimethoxysilane, methyltrioximesilane, and vinyltrioximesilane. It is recommended that the silane crosslinking agent is used in the crosslinkable silicone composition according to the present invention in an amount sufficient for curing the composition, preferably in an amount of 0.3 to 200 parts by weight per 100 parts by weight of the organopolysiloxane.

The condensation reaction catalyst used herein is suitably an organic tin compound, or organic titanium compound preferably selected from dibutyltin dilaurate, dibutyltin diacetate, tin octenate, dibutyltin dioctate, tin laurate, tetrabutyl titanate, tetrapropyl titanate, and dibutoxy bis(ethylacetoacetate) titanium. It is preferred that the condensation reaction catalyst be used in our crosslinkable silicone composition in an amount sufficient for crosslinking of the composition, preferably in an amount of 0.01 to 5 parts by weight, more preferably 0.05 to 2 parts by weight per 100 parts by weight of the organopolysiloxane.

A filler can be added to our crosslinkable silicone composition either for adjusting its flowability, or for improving the mechanical strength of the obtained crosslinked silicone particles. Examples of such a filler are reinforcing fillers such as precipitated silica, fumed silica, baked silica and fumed titanium oxide; or non-reinforcing fillers like crushed quartz, diatomaceous earth, aluminosilicic acid, ferrous oxide, zinc oxide, calcium oxide. The surfaces of these fillers can be treated with hexamethylsilazane, trimethylchlorosilane, polydimethylsiloxane, polymethylhydridosiloxane, or similar organosilicon compounds used for such purposes in the art.

By means of a cross-linking reaction, our crosslinkable silicone composition is converted to a rubber-like, gel-like or a similar elastomeric cross-linked substance.

The non-crosslinkable silicone oil contained in the cross-linkable composition according to the present invention is used in an amount sufficient for maintaining the crosslinked particles resulting from crosslinking of our crosslinkable composition within the non-cross-linkable silicone oil, i.e. the crosslinked particles are surrounded by droplets of the silicone oil. In other words, it should be used in excess of the quantity of the non-crosslinkable oil that can be held by the crosslinked product for example by swelling. Thus the crosslinked silicone particles are swollen and surrounded by the non-crosslinkable silicone oil. The amount that can be held depends on the combination of the crosslinkable species and the non-crosslinkable silicone oil in the silicone compositions. The relative amounts of crosslinkable species and non-crosslinkable silicone oil to achieve the above result can be easily established by the person skilled in the art by routine experimentation. Preferably the non-cross-linkable silicone oil should be used in an amount within a range of 200 to 5,000 parts by weight, more preferred 250 to 2,000 parts by weight per 100 parts by weight of the crosslinkable silicon species of the silicone composition.

The method for preparation of the silicone dispersion of the present invention comprises dispersing a crosslinkable silicone composition that contains the non-cross-linkable silicone oil as described above in water, and then conducting a cross-linking reaction. Dispersing the cross-linkable silicone composition in water can be carried out with the use of a homomixer, paddle mixer, Henschel™ mixer, homodisperser, colloid mixer, propeller stirrer, homodisperser, homogenizer, in-line continuous emulsifier, ultrasound emulsifier, vacuum kneader, or any other device known in the art for dispersing compositions in water.

There are no special limitations with regard to the amount of water used in the method of the present invention. It is preferred, however, that water is used in an amount of 5 to 99 wt.%, preferably 10 to 80 wt.% based upon the total weight of the dispersion.

If it is desired to improve the stability of the cross-linkable silicone composition in water or to ensure dispersion, the composition may comprise a surfactant selected from nonionic, cationic, or anionic surface-active agents. The most preferred surfactant is a nonionic surface-active agent. It is recommended that the surfactant is used in an amount of 0.1 to 20 parts by weight, preferably 0.5 to 10 parts by weight per 100 parts by weight of the crosslinkable silicone composition.

The dispersion of our crosslinkable silicone composition in water is then either heated, maintained at room temperature, or irradiated with high-energy rays, whereby a cross-linking reaction is affected.

It is most preferred that silicone oil droplets dispersed in water have an average diameter within a range of 0.1 to 500 µm, preferably 0.5 to 200 µm. If an average diameter of droplets is below the lower limit or exceeds the upper limit, the dispersion will tend to lose its stability.

The following is a more detailed description of the method used for the preparation of the silicone composition of the present invention. The method comprises the removal of water from the silicone dispersion of the present invention that contains crosslinked silicone particles in silicone oil droplets uniformly dispersed in water. Preferably water is removed from the dispersion by air drying, hot-air drying, vacuum drying, or heating. A silicone composition obtained by this method comprises a dispersion of cross-linked silicone particles uniformly dispersed in silicone oil. This composition can be prepared in a liquid, cream-like, paste-like, or a grease-like form. The composition can be used as a lubricating agent or a cosmetic substance.

### EXAMPLES

The silicone dispersions of the invention, methods of their preparation, and methods for the preparation of the silicone compositions of the invention will now be considered in more detail with reference to the following practical examples. In these examples, all values of viscosity were measured at 25°C. The following procedures were used for determining the average diameter of silicone oil droplets, the average diameter of crosslinked silicone particles, and various characteristics of the silicone composition.

### Average Diameter of Silicone Oil Droplets

The diameter of silicone oil droplets was measured with the use of a laser-diffraction type particle distribution measurement instrument of Horiba Seisakusho Co., Model LA-500. The obtained median diameter (a droplet diameter corresponding to 50% of the accumulated distribution) was defined as an average droplet diameter.

### Stability of the Silicone Dispersion

180 mL of the silicone dispersion was sealed in a 225 mL glass bottle 105 mm long and 50 mm in diameter nd kept for 1 week at room temperature. The thickness of a water layer which had been separated from the silicone dispersion was then measured.

### Dispersibility of Cross-Linked Silicone Particles

A silicone dispersion was dried in air on a glass plate, and then the shape, aggregation, and distribution of cross-linked silicone particles were observed under a stereoscopic microscope. Structures with a dispersion of primary particles were designated by a symbol "O", structures with aggregated particles with the size of about several hundred micrometers and primary particles exceeding 500 µm in size were designated by a symbol "x", and structures with particles having intermediate dimensions between the aforementioned categories were designated by a symbol "△".

### Average Diameter of Cross-Linked Silicone Particles

The silicone dispersion was dried in air on a glass plate, and then a sample was prepared under a stereoscopic microscope by accumulating the cross-linked particles. The collected sample was then observed under an electronic microscope, and an average particle diameter was determined based on 10 particles.

### Viscous-Elastic Properties of the Silicone Composition

A storage elasticity coefficient G' (x10³ dyne/cm²), loss elasticity coefficient G'' (x10 dyne/cm²), and loss *tan* δ were measured by means of an ARES™ viscosimeter of Reometric Scientific Co., Inc. Measurements were carried out under the following conditions: room temperature; 25 mm parallel plates; 0.5 to 0.6 mm gap; 10% strain; 0.1 to 50 rad/s oscillation frequency.

### Practical Example 1

A cross-linkable silicone composition was prepared by mixing the following components: 18.8 parts by weight of a dimethylpolysiloxane having 400 mPa·s viscosity with terminal dimethylvinylsiloxy groups; 1.2 parts by weight of a copolymer of methylhydridosiloxane and dimethylsiloxane having terminal trimethylsiloxy groups having a viscosity of 30 mPa·s and a content of silicon-bonded hydrogen atoms of 0.5 wt.%; and 80 parts by weight a dimethylpolysiloxane having terminal trimethylsiloxy groups and a viscosity of 100 mPa·s. The obtained cross-linkable silicone composition was then combined with 53 parts by weight of a 3 wt.% aqueous solution of polyoxyethylene nonylphenylether (HLB = 13.1). After emulsification, 50 parts by weight of pure water were added to the mixture. As a result, an aqueous dispersion of a cross-linkable silicone composition was prepared.

A platinum catalyst comprising a complex of platinum and 1,1-divinyl-1,1,3,3-tetramethoxydisiloxane as its main component with an average diameter of the platinum catalyst particles equal to 0.05 µm and concentration of metallic platinum of 0.05 wt.% was added to the aforementioned aqueous dispersion of the cross-linkable silicone composition, and the components were uniformly mixed so that the contents of the metallic platinum in weight units became equal to 20 ppm. As a result, an aqueous dispersion of a cross-linkable silicone composition containing a hydrosylilation catalyst was obtained.

The obtained dispersion was kept intact at room temperature for one day, and then the cross-linkable silicone composition was subjected to a hydrosylilation reaction, whereby a silicone dispersion having cross-linked silicone particles in droplets of silicone oil dispersed in water was obtained.

The dispersion was then transferred to a 5 cm diameter aluminum plate, and water was removed from the dispersion by drying in air, while maintaining an air draft for 3 days. As a result, a silicone composition consisting of a silicone oil and silicone particles was prepared. This silicone composition had a cream-like form. Observation of the composition under a stereoscopic microscope showed that it consisted of cross-linked silicone particles uniformly dispersed within the silicone oil. The cross-linked silicone particles were spherical in shape.

### Practical Example 2

An aqueous dispersion of a cross-linked silicone composition was prepared as in Practical Example 1, with the exception that 80 parts by weight of a dimethylpolysiloxane having terminal trimethylsiloxy groups and a viscosity of 6 mPa·s instead of 100 mPa·s as for the dimethypolysiloxane used in Practical Example 1. Next the dispersion was subjected to a hydrosylilation reaction, whereby a silicone dispersion having cross-linked silicone particles contained within silicone oil droplets uniformly dispersed in water was obtained.

A silicone composition consisting of a silicone oil and cross-linked silicone particles was prepared by removing water from the aforementioned dispersion in the same manner as in Practical Example 1. The obtained composition had a cream-like appearance. Observation of the composition under a stereoscopic microscope showed that it consisted of cross-linked silicone particles uniformly dispersed in the silicone oil. The cross-linked silicone particles were spherical in shape.

### Practical Example 3

A mixture was prepared by uniformly mixing the following components: 100 parts by weight of a dimethylpolysiloxane having a viscosity of 1,000 mPa·s and terminal hydroxyl groups resulting in a hydroxyl content of 1.3 wt.%, 4.83 parts by weight of a methylhydridopolysiloxane having terminal trimethylsiloxy groups and a viscosity of 10 mPa·s with a content of silicon-bonded hydrogen atoms of 1.5 wt.%; 500 parts by weight of a dimethylpolysiloxane having terminal trimethylsiloxy groups and a viscosity of 1000 mPa·s; and 0.75 parts by weight of dibutyltin dioctate catalyst. The composition was then combined with a mixture consisting of 5 wt.% of Tergitol™ TMN-6, a product of Union Carbide Co., which is an ethyleneoxide adduct of trimethylnonanole; and 80 parts by weight of ion-exchange water. The obtained product was emulsified and then combined with 35 parts by weight of pure water. As a result, an aqueous dispersion of a cross-linkable silicone composition was obtained.

A portion of the resulting dispersion was sprayed with a spray drier with an input temperature of 300°C and an output temperature of 100°C. A grease-like substance was accumulated on the inner walls of the drier, so it was impossible to obtain any cross-linked silicone particles.

After subjecting the crosslinkable silicone composition to a condensation reaction by keeping the aforementioned aqueous dispersion of the crosslinkable silicone composition intact for 1 week at room temperature, a silicone dispersion containing crosslinked silicone particles within silicone oil droplets uniformly dispersed in water was obtained.

A silicone composition consisting of a silicone oil and cross-linked silicone particles was prepared by removing water from the aforementioned dispersion in the same manner as in Practical Example 1. The obtained composition had a cream-like appearance. Observation of the composition under a general-purpose microscope showed that it consisted of crosslinked silicone particles uniformly dispersed in the silicone oil. The cross-linked silicone particles were spherical in shape.

### Comparative Example 1

A mixture "A" was prepared by uniformly mixing the following components: 100 parts by weight of a dimethylpolysiloxane having a viscosity of 1,000 mPa·s and terminal hydroxyl groups with a content of hydroxyl groups of 1.3 wt.%; 10 parts by weight of a methylhydridopolysiloxane having terminal trimethylsiloxy groups and a viscosity of 10 mPa·s with a content of silicon-bonded hydrogenatoms of 1.5 wt.%; and 50 parts by weight of a dimethylpolysiloxane having terminal trimethylsiloxy groups and a viscosity of 1000 mPa·s.

A mixture "B" was prepared by uniformly mixing the following components: 100 parts by weight of a dimethylpolysiloxane having a viscosity of 1,000 mPa·s and terminal hydroxyl groups with a content of hydroxyl groups of 1.3 wt.%;
50 parts by weight of a dimethylpolysiloxane having terminal trimethylsiloxy groups and a viscosity of 1000 mPa·s; and 1.5 parts by weight of dibutyltindioctate.

After combining mixture "A" and mixture "B" in a 1:1 ratio, the product was further combined with a mixture consisting of 5 wt.% of Tergitol™ TMN-6 and 1700 parts by weight of ion-exchange water. The obtained product was emulsified. After removal of water, cross-linked silicone particles were obtained with a 98% yield. The obtained particles had rubber-like properties and were of a spherical shape. However, undesirable bleeding of silicone oil from these crosslinked silicone particles was observed.

### Comparative Example 2

A crosslinkable silicone composition was prepared by mixing the following components: 44.5 parts by weight of a dimethylpolysiloxane having a viscosity of 5 mPa·s and terminal vinylmethylsiloxy groups; 100 parts by weight of a methylhydridopolysiloxane with a content of silicon-bonded hydrogen atoms of 1.5 wt.% having a viscosity of 20 mPa·s; and 758 parts by weight of a dimethylpolysiloxane having terminal trimethylsiloxy groups and a viscosity of 6 mPa·s and a content of silicon-bonded hydrogen atoms of 1.5 wt.%; and 50 parts by weight of a dimethylpolysiloxane having terminal trimethylsiloxy groups and a viscosity of 1000 mPa·s. A cross-linkable silicone composition was then prepared by adding 0.5 parts by weight of a 2 wt.% isopropanol solution of chloroplatinic acid. The obtained cross-linkable silicone composition was heated to 70-80°C. stirred for 2 hours, whereby hydrosylilation was performed. A gelatinous silicone composition was produced. This composition was kneaded between three rollers under shearing conditions to produce a paste-like silicone composition. Observations under a general-purpose microscope showed that the silicone particles dispersed in the silicone oil had an irregular shape, the dispersion was non-uniform, and the diameter of the cross-linked silicone particles was within a range of 100 to 500 µm.

**TABLE 1**

| Type Item | Present Invention | | | Comp. Examples | |
|---|---|---|---|---|---|
| | Pr. Ex. 1 | Pr. Ex. 2 | Pr. Ex. 3 | Comp. Ex. 1 | Comp. Ex. 2 |
| Average Silicone Oil Droplet Diameter (µm) | 8 | 7 | 5 | 5 | - |
| Stability (mm) | 0 | 0 | 5 | 49 | - |
| Dispersibility of Crosslinked Silicone Particles | O | O | Δ | X | - |
| Average Particle Crosslinked Silicone Diameter (µm) | 3 | 3 | 2 | 5 | 17 |
| Silicone Composition | | | | | |
| 1 rad/s | 9.7 | 15 | 0.15 | | 5.0 |
| G' | | | | | |
| 10 rad/s | 13 | 37 | 0.43 | - | 7.5 |
| | | | | | |
| 1 rad/s | 6.3 | 16 | 0.40 | - | 4.7 |
| G" | | | | | |
| 10 rad/s | 11 | 21 | 2.2 | - | 4.0 |
| | | | | | |
| 1 rad/s | 0.21 | 1.1 | 2.6 | - | 0.82 |
| tan δ | | | | | |
| 10 rad/s | 0.29 | 0.55 | 5.2 | - | 0.58 |

*Because of the physical characteristics of cross-linked silicone particles, measurements were impossible in Comparative Example 1.

### Comparative Example 3

A crosslinkable silicone composition was prepared by mixing 94 parts by weight of a dimethylpolysiloxane having terminal dimethylyinylsiloxy groups and a viscosity of 400 mPa·s, and 6 parts by weight of copolymer of methylhydridosiloxane and dimethylsiloxane having terminal trimethylsiloxy groups with a content of silicon-bonded hydrogen atoms of 0.5 wt.% and a viscosity of 30 mPa·s. This mixture was then combined with 53 parts by weight of a 3 wt.% aqueous solution of polyoxyethylene nonylphenylether (HLB = 13.1). The mixture was emulsified and combined with 50 parts by weight of pure water. An aqueous emulsion of a cross-linkable silicone composition was thereby produced.

The aforementioned aqueous emulsion was uniformly mixed with a separately prepared aqueous emulsion of a platinum-type catalyst having a 1,1-divinyl-1,1,3,3-tetramethoxydisiloxane complex platinum as its main component with an average particle diameter of the platinum-type catalyst equal to 0.05 µm; and a concentration of metallic platinum of 0.05 wt.%, so that the amount of metallic platinum in the aforementioned catalyst was 20 ppm based on the amount of the dimethylpolysiloxane. An aqueous dispersion of a crosslinkable silicone composition was thus prepared.

The obtained dispersion was kept intact at room temperature for one day, and then the crosslinkable silicone composition was subjected to a hydrosylilation reaction, whereby an aqueous suspension of cross-linked silicone particles dispersed in water was prepared. The suspension was combined with an aqueous emulsion of a 50 wt.% concentration of a dimethylpolysiloxane with terminal trimethylsiloxy groups having a viscosity of 100 mPa·s,. The amount of the emulsion was four times the amount of crosslinked silicone particles in the suspension. In the resulting mixture the crosslinked silicone particles were not uniformly contained in silicone oil droplets. The mixture was then transferred to a 5 cm diameter aluminum plate, and water was removed from the dispersion by drying in air, while maintaining an air draft for 3 days. As a result, a silicone composition consisting of a silicone oil and silicone particles was prepared. This silicone composition had a creamy consistency, but the silicone oil was observed floating on the surface of the composition. Observation of the composition under a stereoscopic microscope showed that cross-linked silicone particles were dispersed non-uniformly in the silicone oil. The crosslinked silicone particles were spherical in shape.

Comparative Example 1 and 2 show that if a silicone composition is not made by starting from a silicone dispersion having crosslinked silicone particles contained in silicone oil droplets dispersed in water as required by the present invention the desired stability and uniformity of the dispersion of crosslinked silicone particles in silicone oil can not be achieved.

From Comparative Example 3 it is evident that if silicone oil is added after formation of crosslinked silicone particles dispersed in water the aqueous silicone dispersion wherein the crosslinked silicone particles are contained in silicone oil droplets of the present invention can not be obtained and as a result the desired uniform dispersion of crosslinked silicone particles in silocone oil can not be achieved upon removal of water.

## Claims

1. A silicone dispersion comprising crosslinked silicone particles which have an average particle diameter of 0.1 to 100 µm, the crosslinked silicone particles being contained in silicone oil droplets having an average diameter of more than 0.1 to 500 µm, the silicone oil droplets containing the crosslinked silicone particles being dispersed in water, the diameter of the crosslinked silicone particles being smaller than the diameter of the silicone oil droplets.

2. The silicone dispersion of claim 1 wherein the amount of silicone oil is within the range of 200-5000 parts by weight per 100 parts by weight of the crosslinked silicone particles.

3. A method of manufacturing a silicone dispersion containing crosslinked silicone particles of an average particle diameter of 0.1 to 100 µm in silicone oil droplets of an average droplet diameter of more than 0.1 to 500 µm and wherein the diameter of the crosslinked silicone particles is smaller than the diameter of the silicone oil droplets, the method comprising:
a) dispersing in water a crosslinkable silicone composition containing crosslinkable silicon species and a non-crosslinkable silicone oil in an amount which exceeds the amount of non-crosslinkable silicone oil which can be held by the crosslinked product resulting from the crosslinkable silicon species; and
b) affecting crosslinking of the crosslinkable silicone composition.

4. The method of claim 3 wherein the amount of silicone oil is within the range of 200-5000 parts by weight per 100 parts by weight of the crosslinkable silicon species.

5. The method of any of claim 3 or 4 wherein the crosslinkable silicone composition is crosslinked by a hydrosilylation or a condensation reaction.

6. A method for the preparation of a silicone composition containing cross-linked silicone particles with an average diameter of 0.1 to 100 µm which are uniformly dispersed in a silicone oil, the method comprising removing water from a silicone dispersion according to any of claims 1 or 2 or obtainable from the method according to any of claims 3 to 5.

7. A silicone composition containing cross-linked silicone particles with an average diameter of 0.1 to 100 µm which are uniformly dispersed in a silicone oil obtainable from the method according to claim 6.

8. A lubricating agent comprising a silicone composition according to claim 7.

9. A cosmetic formulation comprising a silicone composition according to claim 7.

## Patentansprüche

1. Silicondispersion enthaltend vernetzte Siliconteilchen, die einen mittleren Teilchendurchmesser von 0,1 bis 100 µm aufweisen, wobei die vernetzten Siliconteilchen sich in Siliconöltropfen mit einem mittleren Durchmesser von mehr als 0,1 bis 500 µm befinden, die Siliconöltropfen, die die vernetzten Siliconteilchen enthalten, in Wasser dispergiert sind und der Durchmesser der vernetzten Siliconteilchen kleiner ist als der Durchmesser der Siliconöltropfen.

2. Silicondispersion nach Anspruch 1, wobei die Menge an Siliconöl innerhalb eines Bereichs von 200 bis 5000 Gewichtsteilen pro 100 Gewichtsteilen der vernetzten Siliconteilchen liegt.

3. Verfahren zur Herstellung einer Silicondispersion enthaltend vernetzte Siliconteilchen mit einem mittleren Teilchendurchmesser von 0,1 bis 100 µm in Siliconöltropfen eines mittleren Tropfendurchmessers von mehr als 0,1 bis 500 µm, wobei der Durchmesser der vernetzten Siliconteilchen geringer ist als der Durchmesser der Siliconöltropfen und das Verfahren umfasst:
(a) Dispergieren einer vernetzbaren Siliconzusammensetzung, die vernetzbare Siliciumspezies und ein nicht vernetzbares Siliconöl in einer Menge enthält, die die Menge an nicht vernetzbarem Siliconöl, die durch das vernetzte Produkt resultierend aus den vernetzbaren Siliciumspezies gehalten werden kann, übersteigt, in Wasser und
(b) Bewirken der Vernetzung der vernetzbaren Siliconzusammensetzung.

4. Verfahren nach Anspruch 3, wobei die Menge an Siliconöl innerhalb eines Bereichs von 200 bis 5000 Gewichtsteilen pro 100 Gewichtsteilen der vernetzbaren Siliciumspezies liegt.

5. Verfahren nach einem der Ansprüche 3 oder 4, wobei die vernetzbare Siliconzusammensetzung durch eine Hydrolysierungs- oder eine Kondensationsreaktion vernetzt wird.

6. Verfahren zur Herstellung einer Siliconzusammensetzung, die vernetzte Siliconteilchen mit einem mittleren Durchmesser von 0,1 bis 100 µm enthält, die gleichförmig in einem Siliconöl dispergiert sind, wobei das Verfahren das Entfernen von Wasser aus einer Silicondispersion nach einem der Ansprüche 1 oder 2 oder erhältlich durch das Verfahren nach einem der Ansprüche 3 bis 5 umfasst.

7. Siliconzusammensetzung enthaltend vernetzte Siliconteilchen mit einem mittleren Durchmesser von 0,1 bis 100 µm, die einheitlich in einem Siliconöl dispergiert sind, erhältlich nach dem Verfahren nach Anspruch 6.

8. Ein Gleitmittel enthaltend eine Siliconzusammensetzung nach Anspruch 7.

9. Eine kosmetische Formulierung enthaltend eine Siliconzusammensetzung nach Anspruch 7.

## Revendications

1. Dispersion de silicone comprenant des particules de silicone réticulée ayant un diamètre moyen de particules de 0,1 à 100 µm, les particules de silicone réticulée étant contenues dans des gouttelettes d'huile de silicone ayant un diamètre moyen de plus de 0,1 à 500 µm, les gouttelettes d'huile de silicone contenant les particules de silicone réticulée étant dispersées dans de l'eau, le diamètre des particules de silicone réticulée étant inférieur au diamètre des gouttelettes d'huile de silicone.

2. Dispersion de silicone selon la revendication 1, dans laquelle la quantité d'huile de silicone est située dans l'intervalle de 200 à 5000 parties en masse pour 100 parties en masse de particules de silicone réticulée.

3. Méthode de fabrication d'une dispersion de silicone comprenant des particules de silicone réticulée ayant un diamètre moyen de particules de 0,1 à 100 µm dans des gouttelettes d'huile de silicone ayant un diamètre moyen de gouttelettes de plus de 0,1 à 500 µm, le diamètre des particules de silicone réticulée étant inférieur au diamètre des gouttelettes d'huile de silicone, la méthode comprenant :
c) la dispersion dans l'eau d'une composition de silicone réticulable contenant des espèces de silicium réticulable et d'une huile de silicone non réticulable en une quantité dépassant la quantité de l'huile de silicone non réticulable qui peut être retenue par le produit réticulé résultant des espèces de silicium réticulables et
d) la réalisation de la réticulation de la composition de silicone réticulable.

4. Méthode selon la revendication 3, dans laquelle la quantité d'huile de silicone est située dans l'intervalle de 200 à 5000 parties en masse pour 100 parties en masse d'espèces de silicone réticulables.

5. Méthode selon l'une quelconque des revendications 3 ou 4, dans laquelle la composition de silicone réticulable est réticulée par une réaction d'hydrosilylation ou de condensation.

6. Méthode pour la préparation d'une composition de silicone contenant des particules de silicone réticulée ayant un diamètre moyen de 0,1 à 100 µm uniformément dispersées dans une huile de silicone, la méthode comprenant l'élimination de l'eau d'une dispersion de silicone selon l'une quelconque des revendications 1 ou 2 ou pouvant être obtenue par la méthode selon l'une quelconque des revendications 3 à 5.

7. Composition de silicone contenant des particules de silicone réticulée ayant un diamètre moyen de 0,1 à 100 µm uniformément dispersées dans une huile de silicone pouvant être obtenue par la méthode selon la revendication 6.

8. Agent lubrifiant comprenant une composition de silicone selon la revendication 7.

9. Formulation cosmétique comprenant une composition de silicone selon la revendication 7.
